# EUROPEAN PATENT APPLICATION

(11) **EP 4 248 962 A1**
(43) Date of publication of application: **27.09.2023**
(21) Application number: 21894757.0
(22) Date of filing: 19.11.2021
(51) Int. Cl.: A61K 31/216, A61K 9/10, A61K 47/38, A61K 47/40, A61P 27/02

(54) **EYEDROPS FOR AMELIORATING OR PREVENTING RETINAL CIRCULATORY DISTURBANCE AND DISORDERS ASSOCIATED WITH RETINAL NERVE BLOOD VESSELS**

(30) Priority: 19.11.2020 JP 2020192736
(71) Applicant: Nihon University, Tokyo 102-8275 (JP); Kinki University, Higashi-Osaka-shi Osaka 577-8502 (JP)
(72) Inventor: NAGAOKA Taiji, Tokyo 102-8275 (JP); NAGAI Noriaki, Higashiosaka-shi, Osaka 577-8502 (JP); YOKOTA Harumasa, Tokyo 102-8275 (JP); YAMAGAMI Satoru, Tokyo 102-8275 (JP); HANAGURI Junya, Tokyo 102-8275 (JP); OTAKE Hiroko, Higashiosaka-shi, Osaka 577-8502 (JP)
(74) Representative: Nederlandsch Octrooibureau
(86) International application number: PCT/JP2021/042628
(87) International publication number: WO 2022/107886

(57) **Abstract**

Eyedrops for ameliorating retinal circulation disorder and retinal neurovascular coupling disorder, the eyedrops containing fibrate-containing nanoparticles.

## Description

### [Technical Field]

The present invention relates to eyedrops for ameliorating or preventing retinal circulation disorder and retinal neurovascular coupling disorder.

Priority is claimed on Japanese Patent Application No. 2020-192736, filed on November 19, 2020, the content of which is incorporated herein by reference.

### [Background Art]

Fibrates are known to have a retinal neovascularization suppressing effect in a case of being taken orally. For example, Patent Document 1 reports that retinal neovascularization was suppressed by gavage administration of a fenofibrate to mice. However, side effects (rhabdomyolysis and the like) caused by fibrates used in combination with statins have been reported, and in actual clinical practice, the fibrates are not used for the purpose of treating ocular diseases.

As a method for administering a drug for ocular diseases, topical administration is preferable to oral administration since it has fewer systemic side effects. For example, an anti-VEGF agent widely used for treatment of retinal diseases such as age-related macular degeneration and diabetic macular edema is mainly administered through intravitreal administration. However, vitreous hemorrhage, lens damage, and the like have been reported, and serious ocular complications such as endophthalmitis have also been reported. From the viewpoint of safety, administration by eyedrops is desirable. However, it is difficult for an effective concentration of a drug to reach the retinal tissues present in the posterior portion of the eyeball through administration by eyedrops commonly used.

On the other hand, retinal circulation disorder and retinal neurovascular coupling disorder occur earlier than obvious retinopathy findings, and are considered to contribute the development of retinal diseases. However, eyedrops which are effective in ameliorating these disorders have not been put into practical use.

### [Citation List]

### [Patent Document]

[Patent Document 1]
Published Japanese Translation No. 2016-539098 of the PCT International Publication

### [Summary of Invention]

### [Technical Problem]

In a case where retinal circulation disorder or retinal neuro coupling disorder. can be ameliorated or prevented by eyedrops, retinal diseases caused by these disorders can be prevented or treated safely and effectively.

Accordingly, an object of the present invention is to provide an eyedrop capable of ameliorating or preventing retinal circulation disorder and retinal neurovascular coupling disorder.

### [Solution to Problem]

The present invention includes the following aspects.
[1] An eyedrop for ameliorating or preventing retinal circulation disorder and retinal neurovascular coupling disorder, the eyedrop containing nanoparticles including a fibrate.
[2] The eyedrop as described in [1], in which wherein the nanoparticles are a wet-ground product of a fibrate.
[3] The eyedrop as described in [1] or [2], further containing a thickener.
[4] The eyedrop as described in [3], in which the thickener is a cellulose-based thickener.
[5] The eyedrop as described in [4], in which the cellulose-based thickener is methylcellulose.
[6] The eyedrop as described in any one of [1] to [5], further containing cyclodextrins.
[7] The eyedrop as described in [6], in which the cyclodextrins are 2-hydroxypropyl-β-cyclodextrin.

### [Advantageous Effects of Invention]

According to the present invention, there is provided an eyedrop capable of ameliorating or preventing retinal circulation disorder and retinal neurovascular coupling disorder.

### [Brief Description of Drawings]

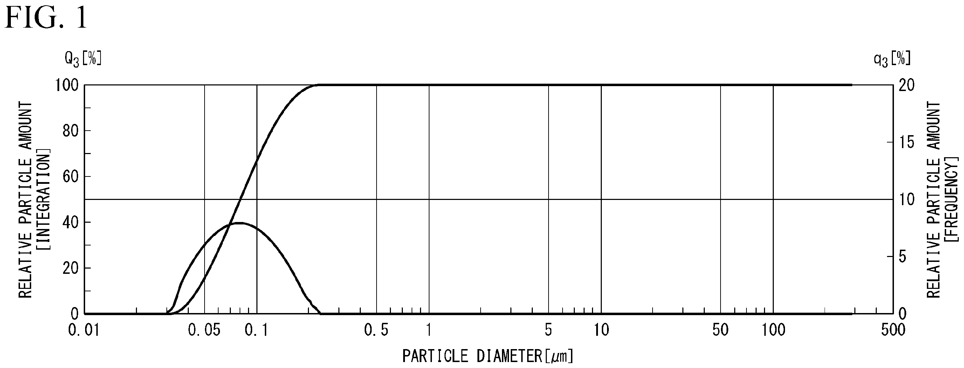

FIG. 1 shows the results of particle size distribution analysis by a laser diffraction/scattering method of fibrate nanoparticles prepared in Examples.

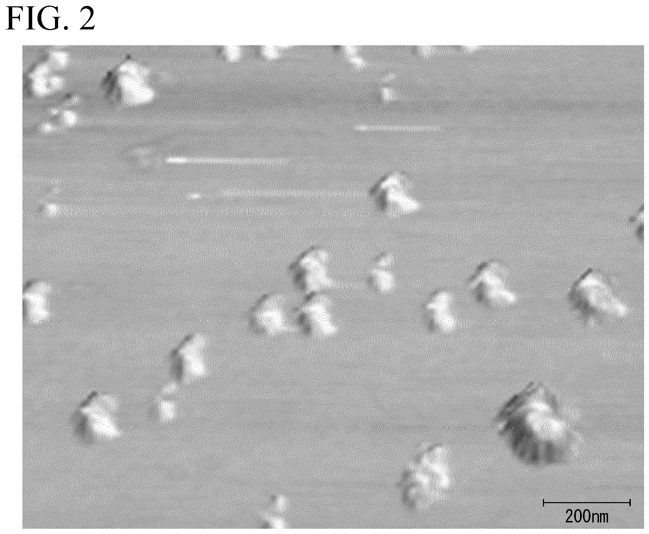

FIG. 2 shows an atomic force microscope image of fibrate nanoparticles prepared in Examples.

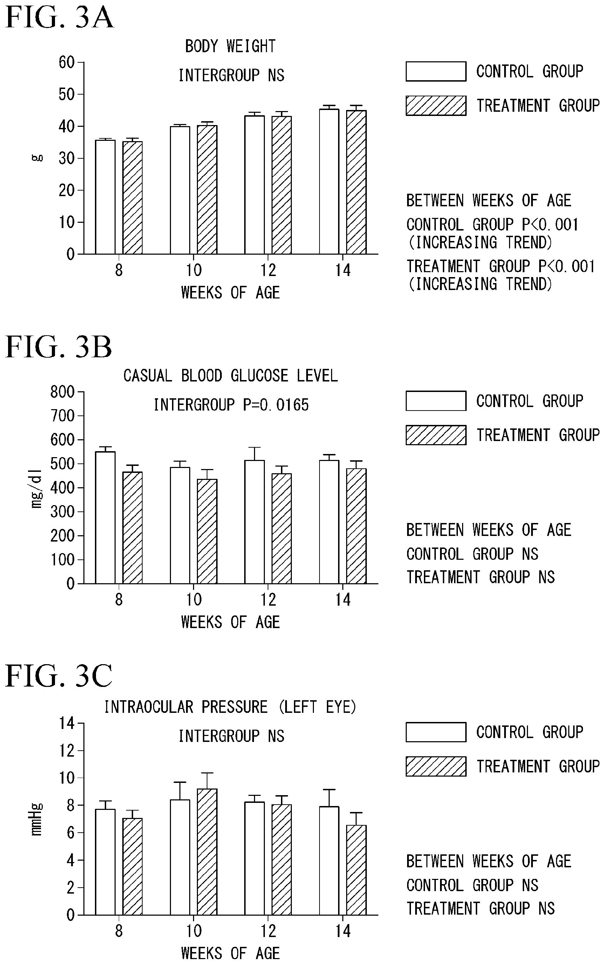

FIG. 3A shows a temporal change in body weight of diabetic model mice which have been subjected to an eyedrop administration test. Mean ± SEM. Treatment group: Administered with a fibrate nanoparticle-containing eyedrop (n = 6). Control group: Administered with a vehicle nanoparticle eyedrop (n = 6). NS: No significant difference.
FIG. 3B shows a temporal change in casual blood glucose level at any time in diabetic model mice which have been subjected to an eyedrop administration test. Mean ± SEM. Treatment group: Administered with a fibrate nanoparticle-containing eyedrop (n = 6). Control group: Administered with a vehicle nanoparticle eyedrop (n = 6). NS: No significant difference.
FIG. 3C shows a temporal change in intraocular pressure (left eye) of diabetic model mice which have been subjected to an eyedrop administration test. Mean ± SEM. Treatment group: Administered with fibrate nanoparticle-containing eyedrop (n = 6). Control group: Administered with vehicle nanoparticle eyedrop (n = 6). NS: No significant difference.

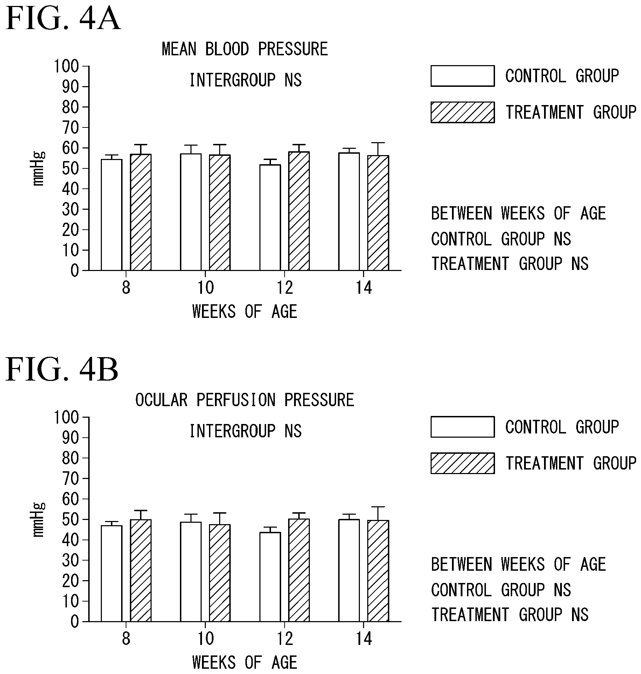

FIG. 4A shows a temporal change in mean blood pressure of diabetic model mice which have been subjected to an eyedrop administration test. Mean ± SEM. Treatment group: Administered with fibrate nanoparticle-containing eyedrop (n = 6). Control group: Administered with vehicle nanoparticle eyedrop (n = 6). NS: No significant difference.
FIG. 4B shows a temporal change in ocular perfusion pressure in diabetic model mice which have been subjected to an eyedrop administration test. Mean ± SEM. Treatment group: Administered with a fibrate nanoparticle-containing eyedrop (n = 6). Control group: Administered with a vehicle nanoparticle eyedrop (n = 6). NS: No significant difference.

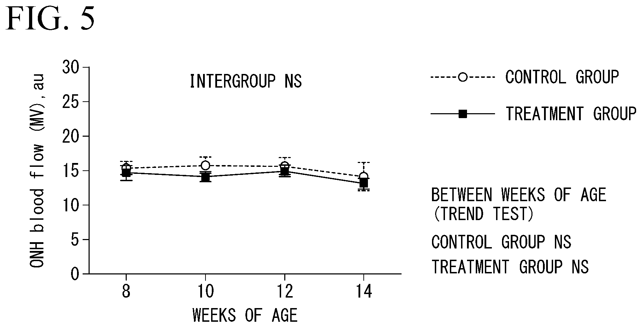

FIG. 5 shows a temporal change in baseline optic nerve head blood flow in diabetic model mice which have been subjected to an eyedrop administration test. Mean ± SEM. Treatment group: Administered with a fibrate nanoparticle-containing eyedrop (n = 6). Control group: Administered with a vehicle nanoparticle eyedrop (n = 6). NS: No significant difference.

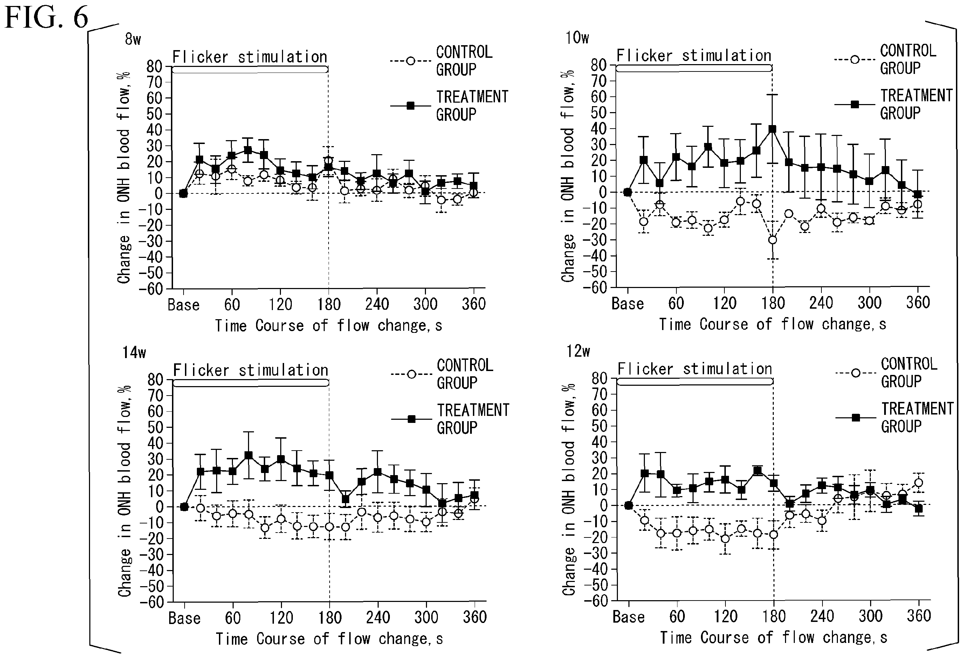

FIG. 6 shows each of changes in ocular blood flow after flicker stimulation in diabetic model mice at 8 weeks, 10 weeks, 14 weeks, and 12 weeks of ages, which have been subjected to an eyedrop administration test. Treatment group: Administered with a fibrate-containing eyedrop (n = 6). Control group: Administered with a vehicle eyedrop (n = 6).

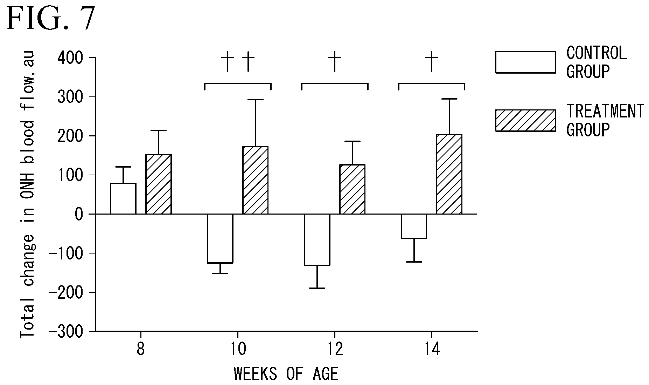

FIG. 7 shows changes in total blood flow after flicker stimulation at each week of age in diabetic model mice which have been subjected to an eyedrop administration test. Treatment group: Administered with a fibrate nanoparticle-containing eyedrop (n = 6). Control group: Administered with a vehicle nanoparticle eyedrop (n = 6).

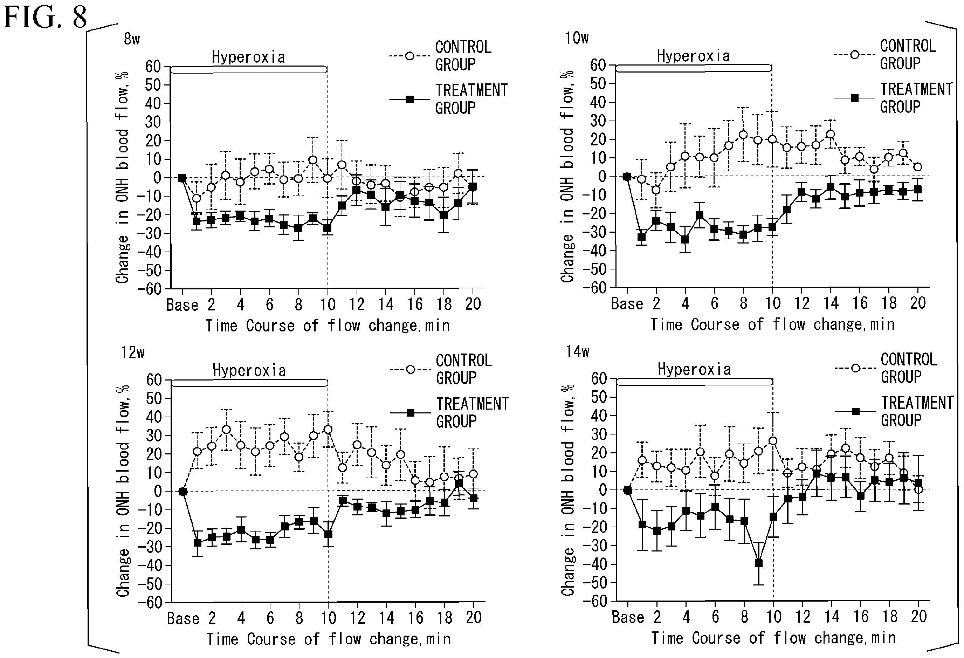

FIG. 8 shows changes in ocular blood flow after hyperoxia stimulation of diabetic model mice at 8 weeks, 10 weeks, 14 weeks, and 12 weeks of ages, which have been subjected to an eyedrop administration test. Treatment group: Administered with a fibrate-containing eyedrop (n = 6). Control group: Administered with a vehicle eyedrop (n = 6).

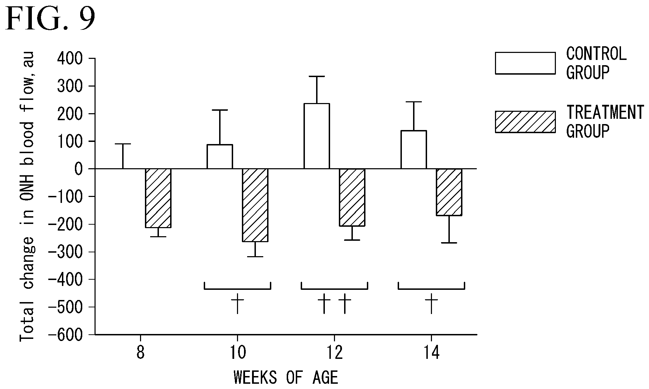

FIG. 9 shows changes in total blood flow after hyperoxia stimulation of diabetic model mice at each week of age, which have been subjected to an eyedrop administration test. Treatment group: Administered with a fibrate nanoparticle-containing eyedrop (n = 6). Control group: Administered with a vehicle nanoparticle eyedrop (n = 6).

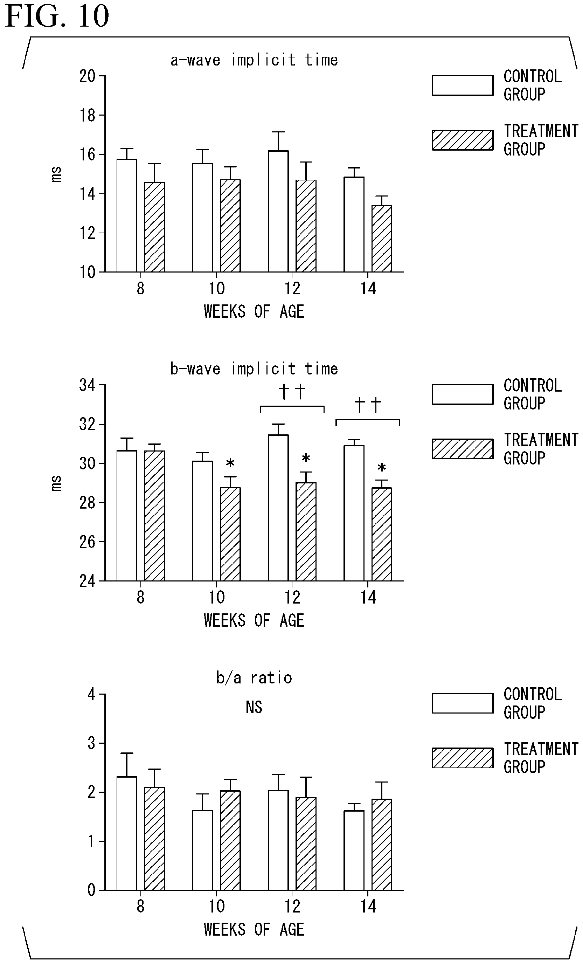

FIG. 10 shows the results of evaluation of a-wave implicit time and b-wave implicit time in electroretinograms of diabetic model mice which have been subjected to an eyedrop administration test. Treatment group: Administered with a fibrate nanoparticle-containing eyedrop (n = 6). Control group: Administered with a vehicle nanoparticle eyedrop (n = 6).

### [Description of Embodiments]

### [Eyedrops]

In one embodiment, the present invention provides an eyedrop for ameliorating retinal circulation disorder and retinal neurovascular coupling disorder, the eyedrop containing nanoparticles including a fibrate.

The term "retinal circulation" means a blood flow in the retina. The term "retinal circulation disorder" means impaired blood flow in the retina. The term "retinal neurovascular coupling" means changes in blood flow in the retina in response to a nerve activity. The term "retinal neurovascular coupling disorder" mean a disturbed reaction of changes in blood flow in response to a nerve activity in the retina.

Occlusion or narrowing of the retinal blood vessels due to thrombus, embolus, and the like causes retinal circulation disorders, which can hinder the supply of oxygen and nutrients to the optic nerve. In addition, retinal neurovascular coupling disorder occur since an increase in retinal blood flow corresponding to an increase in oxygen demand associated with a nerve activity is hindered, causing the retinal tissue to fall into relative hypoxia.

Whether or not retinal circulation disorder and retinal neurovascular coupling disorder are developed can be quantitatively and non-invasively confirmed by measuring a change in retinal blood flow due to flicker stimulation. In an undamaged normal retina, the flicker stimulation usually increases retinal blood flow. However, in a case where retinal circulation disorder and retinal neurovascular coupling disorder occur, an increase in retinal blood flow due to the flicker stimulation is inhibited. Therefore, in a case where the flicker stimulation does not cause the increase in retinal blood flow, it can be evaluated as follows: retinal circulation disorder and retinal neurovascular coupling disorder have occurred. In addition, in a case where an increase in retinal blood flow due to the flicker stimulation can be confirmed from a state where the flicker stimulation does not increase the retinal blood flow, it should be evaluated as follows: retinal circulation disorder and retinal neurovascular coupling disorder are ameliorated.

The retinal circulation disorder and retinal neurovascular coupling disorder contribute to a variety of vitreoretinal diseases. Examples of such vitreoretinal diseases include, but are not limited to, diabetic retinopathy, retinal vein occlusion, retinal artery occlusion, age-related macular degeneration, and central serous chorioretinopathy. Since the eyedrop of the present embodiment can ameliorate the retinal circulation disorder and retinal neurovascular coupling disorder, it can be used for treating or preventing vitreoretinal diseases as described above.

### <Nanoparticles Including Fibrates>

The eyedrop of the present embodiment contains nanoparticles including a fibrate (hereinafter also referred to as "fibrate nanoparticles"). The fibrate nanoparticles are solid nanoparticles, and preferably nanoparticulate fibrate.

The "fibrate" is a generic term for compounds that act as a lipid-lowering drug among amphiphilic carboxylic acid derivatives. Examples of the fibrate include, but are not limited to, gemfibrozil, fenofibrate, bezafibrate, clofibrate, ciprofibrate, beclofibrate, binifibrate, ciprofibrate, clinofibrate, etofibrate, nicofibrate, pirifibrate, ronifibrate, simfibrate, theofibrate, pemafibrate, fibric acid derivatives, and pharmaceutically acceptable salts or esters of the fibric acid derivatives. Among those, fenofibrate is preferable as the fibrate. Fibrates may be used alone or a combination of two or more kinds thereof may be used.

The fibrate content in the eyedrop of the present embodiment is not particularly limited, but may be, for example, 0.1% to 10% (w/v) with respect to the entire eyedrop. The fibrate content is preferably 0.5% to 5% (w/v), more preferably 1% to 4% (w/v), and more preferably 1.5% to 3% (w/v) with respect to the entire eyedrop.

The "nanoparticles" are particles having a particle diameter of 1 nm or more and less than 1,000 nm. The fibrate nanoparticles contained in the eyedrop of the present embodiment preferably have a particle diameter in the range of 10 to 300 nm, and more preferably a particle diameter in the range of 30 to 150 nm from the viewpoint of efficiency of an uptake into cells. Examples of the fibrate nanoparticles include those having a particle size distribution with an average diameter of 50 to 120 nm as measured by a laser diffraction/scattering method or a dynamic light scattering method.

The fibrate nanoparticles can be obtained by wet-grinding the fibrate. The wet grinding of fibrate can be performed using a bead mill, a ball mill, or the like. Dry grinding may be performed before the wet grinding. By performing the dry grinding before the wet grinding, the fibrate can be efficiently nanoparticulated. The fibrate nanoparticles can be referred to as a wet-ground product of the fibrate, a bead mill wet-ground product of the fibrate, or a ball mill wet-ground product of the fibrate. The wet-ground fibrate may be a wet-ground product of a dry-ground fibrate, a bead mill wet-ground product of a dry-ground fibrate, or a ball mill wet-ground product of a dry-ground fibrate.

### <Water>

The eyedrop of the present embodiment contains water as a dispersion medium. As water, water at a grade usable for eyedrops, such as purified water, sterilized purified water, water for injection, and distilled water for injection, can be used.

### <Optional Components>

The eyedrop of the present embodiment may contain optional components in addition to the fibrate nanoparticles. Examples of the optional components include a thickener, a preservative, an isotonizing agent, cyclodextrins, a buffer, and a pH adjuster.

### (Thickener)

The eyedrop of the present embodiment preferably includes a thickener. By allowing the eyedrop to contain the thickener, a phenomenon in which during preparation of the fibrate nanoparticles, the fibrate nanoparticles are brought into a meringue-like or cream-like state is suppressed. Thus, a fibrate nanoparticle dispersion liquid in a good state can be obtained.

Examples of thickener include, but are not limited to, cellulose-based thickeners such as methylcellulose, hydroxyethylcellulose, and hydroxypropyl methylcellulose; vinyl-based thickeners such as a carboxyvinyl polymer, a (completely or partially saponified) polyvinyl alcohol, a polyvinylpyrrolidone; and sodium alginate, sodium chondroitin sulfate, and macrogol.

Among these, the thickener is preferably the cellulose-based thickener, and more preferably methylcellulose since it improves retention of the fibrate nanoparticles and enhances intraocular penetration.

The thickeners may be used alone or a combination of two or more kinds thereof may be used.

The content of the thickener in the eyedrop of the present embodiment is not particularly limited, but is, for example, preferably 0.01% to 3% (w/v), more preferably 0.05% to 2% (w/v), and still more preferably 0.1% to 1% (w/v) with respect to the entire eyedrop.

### (Preservative)

The eyedrop of the present embodiment may include a preservative. By allowing the eyedrop to contain the preservative, the preservability of the eyedrop is improved.

Examples of the preservative include, but are not limited to, invert soaps such as benzalkonium chloride, benzethonium chloride, and chlorhexidine gluconate; parabens such as methylparaben, ethylparaben, propylparaben, and butylparaben; and alcohols such as chlorobutanol, phenylethyl alcohol, and benzyl alcohol.

Among these, as the preservative, the invert soaps which are cationic surfactants are preferable, and benzalkonium chloride is more preferable since they are widely used in eyedrops and have a high preservative effect.

The preservatives may be used alone or a combination of two or more kinds thereof may be used.

The content of the preservative in the eyedrop of the present embodiment is not particularly limited, but is preferably 0.0005% to 0.5% (w/v), more preferably 0.001% to 0.1% (w/v), and still more preferably 0.001% to 0.01% (w/v) with respect to the entire eyedrop.

### (Isotonizing Agent)

The eyedrop of the present embodiment may include an isotonizing agent. By allowing the eyedrop to contain the isotonizing agent, eye irritation can be reduced.

Examples of the isotonizing agent include, but are not limited to, sugars such as glucose; polyhydric alcohols such as propylene glycol, glycerin, mannitol, sorbitol, and xylitol; and inorganic salts such as sodium chloride and potassium chloride.

Among these, the polyhydric alcohols are preferable, and mannitol is more preferable as the isotonizing agent since they can protect the cornea from irritation caused by a protective agent and the like.

The isotonizing agents may be used alone or a combination of two or more kinds thereof may be used.

The content of the isotonizing agent in the eyedrop of the present embodiment is not particularly limited, but is, for example, preferably 0.01% to 3% (w/v), more preferably 0.05% to 2% (w/v), and still more preferably 0.1% to 1% (w/v) with respect to the entire eyedrop.

### (Cyclodextrins)

The eyedrop of the present embodiment preferably contains cyclodextrins. The cyclodextrins can suppress aggregation of fibrate nanoparticles, and uniformly disperse the fibrate nanoparticles.

The term "cyclodextrins" means cyclodextrin or cyclodextrin derivatives. Examples of the cyclodextrins include α-cyclodextrin, β-cyclodextrin, γ-cyclodextrin, and derivatives thereof. Examples of the cyclodextrin derivatives include alkyl derivatives, hydroxyalkyl derivatives, sulfoalkyl ether derivatives, and sugar-bonded derivatives.

Among these, the cyclodextrins are preferably the hydroxyalkyl derivatives, and more preferably 2-hydroxypropyl-β-cyclodextrin since these provide excellent dispersibility of the fibrate nanoparticles.

The cyclodextrins may be used alone or a combination of two or more kinds thereof may be used.

The content of the cyclodextrins in the eyedrop of the present embodiment is not particularly limited, but is, for example, preferably 0.1% to 15% (w/v), more preferably 1% to 10% (w/v), and still more preferably 3% to 8% (w/v) with respect to the entire eyedrop.

### (pH Adjuster)

The eyedrop of the present embodiment may contain a pH adjuster. Examples of the pH adjuster include dilute hydrochloric acid and sodium hydroxide.

### (Buffer)

The eyedrop of the present embodiment may contain a buffer. Examples of the buffer include sodium citrate hydrate, sodium acetate hydrate, sodium hydrogen carbonate, trometamol, boric acid, borax, sodium hydrogen phosphate hydrate, and sodium dihydrogen phosphate.

Examples of the eyedrop of the present embodiment include an eyedrop containing fibrate nanoparticles, water, and a thickener; an eyedrop containing fibrate nanoparticles, water, a thickener, and cyclodextrins; and an eyedrop containing fibrate nanoparticles, water, a thickener, cyclodextrins, and a preservative; and an eyedrop containing fibrate nanoparticles, water, a thickener, cyclodextrins, a preservative, and an isotonizing agent.

More specific examples of the eyedrop include an eyedrop containing fibrate nanoparticles, water, and a cellulose-based thickener; an eyedrop containing fibrate nanoparticles, water, a cellulose-based thickener, and cyclodextrins; an eyedrop containing fibrate nanoparticles, water, a cellulose-based thickener, cyclodextrins, and benzalkonium chloride; and an eyedrop containing fibrate nanoparticles, water, a cellulose-based thickener, cyclodextrins, benzalkonium chloride, and mannitol.

Still more specific examples of the eyedrop include an eyedrop containing fibrate nanoparticles, water, and methylcellulose; an eyedrop containing fibrate nanoparticles, water, methylcellulose, and 2-hydroxypropyl-β-cyclodextrin; an eyedrop containing fibrate nanoparticles, water, methylcellulose, 2-hydroxypropyl-β-cyclodextrin and benzalkonium chloride; and an eyedrop containing fibrate nanoparticles, water, methylcellulose, 2-hydroxypropyl-β-cyclodextrin, benzalkonium chloride, and mannitol.

### <Production Method>

The eyedrop of the present embodiment can be produced by mixing a fibrate with an optional component such as a thickener as appropriate, dispersing the mixture in an aqueous dispersion medium preferably including cyclodextrins, and wet grinding the mixture. Dry grinding may be performed before the wet grinding. The "dry grinding" refers to grinding in a gas. The "wet grinding" refers to grinding in a liquid.

Specific examples of the method for producing an eyedrop of the present embodiment will be described below, but the present invention is not limited thereto.

A thickener and the like are appropriately added to the fibrate, and admixed, followed by mixing and fine grinding. At this time, examples of the components that are miscible with the fibrate include components other than the cyclodextrins and water. These components may be added after the dry grinding and before the wet grinding, but it is preferable that the thickener and the isotonizing agent are blended with the fibrate before the fine grinding. Mixing and fine grinding may first be performed using an agate mortar or the like, and then dry grinding using a bead mill may be performed. Mixing and grinding are preferably performed under low-temperature conditions (for example, 4°C). Zirconia beads having a diameter of about 2 mm can be used for dry grinding by a bead mill.

The finely ground product obtained by dry grinding is dispersed in an aqueous solution including cyclodextrins. At this time, optional components such as a preservative may be added thereto as appropriate. Then, wet grinding is performed using a bead mill or the like. Zirconia beads having a diameter of about 0.1 mm can be used for wet grinding by a bead mill. The wet grinding is preferably performed under low temperature conditions (for example, 4°C). In a case where air bubbles are generated during wet grinding, or in a case where a meringue or cream state is obtained, the state can be returned to a suspension state by centrifuging or the like while adding a small amount of a dispersion medium.

After the wet grinding, the beads are removed and filtering-sterilization was performed using a 0.2 µm filter. As a result, the eyedrop of the present embodiment can be obtained. The concentration of the fibrate nanoparticles in the obtained eyedrop may be adjusted by using a diluent or the like including components other than the fibrate nanoparticles as appropriate at the time of use.

### < Methods of Use>

The eyedrop of the present embodiment can be used to ameliorate retinal circulation disorder and retinal neurovascular coupling disorder. For example, the eyedrop of the present embodiment can be administered to a patient who has or is likely to develop vitreoretinal diseases such as diabetic retinopathy, retinal vein occlusion, retinal artery occlusion, age-related macular degeneration, and central serous chorioretinopathy due to retinal circulation disorders and the retinal neurovascular coupling disorder. By ameliorating the retinal circulation disorders and the retinal neurovascular coupling disorder by the eyedrop of the present embodiment, it is expected to cure, alleviate, or prevent the onset of these vitreoretinal diseases.

The eyedrop of the present embodiment can be used as an eyedrop for treating or preventing vitreoretinal diseases. The vitreoretinal disease to be treated with the eyedrop of the present embodiment may be mild, moderate, or severe. In a case where the vitreoretinal disease is mild, administration of the eyedrop of the present embodiment can be expected to cure the vitreoretinal disease. In a case where the vitreoretinal disease is moderate or severe, the eyedrop of the present embodiment can be used in combination with existing medical treatments (anti-vascular endothelial growth factor VEGF inhibitors and steroids) and surgical treatments (retinal photocoagulation, vitreous surgery, and the like).

The eyedrop of the present embodiment can be used to prevent retinal circulation disorder and retinal neurovascular coupling disorder. It is possible to prevent the occurrence of retinal circulation disorder and retinal neurovascular coupling disorder by prophylactically administering the eyedrop of the present embodiment to a subject at a high risk of developing the retinal circulation disorders and the retinal neurovascular coupling disorder.

The administration interval of the eyedrop of the present embodiment can be appropriately determined depending on a patient's symptom, body weight, age, gender, and the like. The administration interval can be, for example, 2 or 3 times per day.

### [Other Embodiments]

In one embodiment, the present invention provides a method for ameliorating or preventing retinal circulation disorder and retinal neurovascular coupling disorder, the method including administering the eyedrop of the embodiment to a subject.

In one embodiment, the present invention provides a use of nanoparticles including fibrates in the production of an eyedrop for ameliorating or preventing retinal circulation disorder and retinal neurovascular coupling disorder.

In one embodiment, the present invention provides the eyedrop of the embodiment for use in ameliorating or preventing retinal circulation disorder and retinal neurovascular coupling disorder.

In one embodiment, the present invention provides a use of the eyedrop of the embodiment for ameliorating or preventing retinal circulation disorder and retinal neurovascular coupling disorder.

In one embodiment, the present invention provides a method for treating or preventing vitreoretinal diseases, the method including administering the eyedrop of the embodiment to a subject.

In one embodiment, the present invention provides a use of nanoparticles including fibrates in the production of an eyedrop for treating or preventing vitreoretinal diseases.

In one embodiment, the present invention provides the eyedrop of the embodiment for use in treating or preventing vitreoretinal diseases.

In one embodiment, the present invention provides a use of the eyedrop of the embodiment for treating or preventing vitreoretinal diseases.

### [Examples]

The present invention will be described below with reference to Examples, but the present invention is not limited to the following Examples.

### [Preparation of Eyedrops]

### <Preparation of Fibrate Nanoparticle-Containing Eyedrop>

A fibrate-containing eyedrop was prepared by the following procedure.
1) 450 mg of fenofibrate (Teva Takeda Pharma Ltd.), 112.5 mg of methylcellulose, and 112.5 mg of D-mannitol were mixed in an agate mortar and finely ground (at a low temperature (4°C), 1 hour).
2) The finely ground product of 1) was collected and subjected to dry grinding using a bead type cell disruptor (Micro Smash MS-100R, Tomy Seiko Co., Ltd.) (twice with 2 mm zirconia beads, 3,000 rpm, 30 seconds per time) or a multi-specimen cell disruptor (Shake Master Neo, Biomedical Science Co., Ltd.) (with zirconia beads, 500 rpm, 10 minutes) (at a low temperature (4°C)).
3) 450 mg of the dry-ground material obtained in 2) was weighed out and made up to 15 mL with an aqueous 5% (w/v) 2-hydroxypropyl-β-cyclodextrin solution. Then, 75 µL of an aqueous 1% (w/v) benzalkonium chloride (BAC) solution was added thereto.
4) The mixture obtained in 3) was subjected to 30 sets of wet grinding using a bead type cell disruptor (Micro Smash MS-100R, Tomy Seiko Co., Ltd.) (30 times with 0.1 mm zirconia beads, 15,000 rpm, 30 seconds per time, at a low temperature (4°C)). In a case where air bubbles were generated during the wet grinding or a meringue state was formed, the mixture was subjected to centrifugation (800 × g, 60 seconds, 4°C) as appropriate to restore the suspension state.
5) The wet-ground material obtained in 4) was subjected to wet grinding using a multi-specimen cell disruptor (Shake Master Neo, Biomedical Science Co., Ltd.) (with 0.1 mm zirconia beads at 1,500 rpm for 1 hour, at a low temperature (4°C)).
6) The zirconia beads were removed from the ultra-crushed material obtained in 5), and the resultant was filtered and sterilized with a 0.2 µm filter and used as a fibrate-containing eyedrop. The concentration was appropriately adjusted with the following vehicle (dispersion medium) before use, as necessary.

Composition of vehicle.
Benzalkonium chloride 0.005% (w/v)
Mannitol 0.5% (w/v)
Methylcellulose 0.5% (w/v)
2-Hydroxypropyl-β-cyclodextrin 5% (w/v)

A final concentration of each drug in the eyedrop prepared as described above is shown below.
Fenofibrate 2% (w/v)
Benzalkonium chloride 0.005% (w/v)
Mannitol 0.5% (w/v)
Methylcellulose 0.5% (w/v)
2-Hydroxypropyl-β-cyclodextrin 5% (w/v)

### (Particle Size Distribution Analysis by Laser Diffraction/Scattering Method)

The fibrate-containing eyedrop prepared as described above was subjected to particle size distribution analysis using a laser diffraction particle size distribution analyzer (SALD-7100, Shimadzu Corporation). The results are shown in FIG. 1 and Table 1. The fibrate nanoparticles had particle diameters in the range of 34 to 244 nm. FIG. 2 shows an atomic force microscope image of the fibrate nanoparticles.

**[Table 1]**

| | | | | | | | | | | |
|---|---|---|---|---|---|---|---|---|---|---|
| Median diameter (µm) | | | 0.080 | | Average diameter (µm) | | | | 0.080 | |
| Mode diameter (µm) | | | 0.087 | | Standard deviation | | | | 0.192 | |

| 10% diameter (µm) | 20% diameter (µm) | 30% diameter (µm) | 40% diameter (µm) | 50% diameter (µm) | | 60% diameter (µm) | 70% diameter (µm) | 80% diameter (µm) | | 90% diameter (µm) |
|---|---|---|---|---|---|---|---|---|---|---|
| 0.044 | 0.053 | 0.061 | 0.070 | 0.080 | | 0.091 | 0.105 | 0.122 | | 0.146 |

| Particle diameter | Integration % | Frequency % | Particle diameter | Integration % | | Frequency % | Particle diameter | Integration % | | Frequency % |
|---|---|---|---|---|---|---|---|---|---|---|
| × (µm) | Q3 (%) | q3 (%) | × (µm) | Q3 (%) | | q3 (%) | × (µm) | Q3 (%) | | q3 (%) |
| 0.300 | 100.000 | 0.000 | 0.132 | 84.804 | | 6.184 | 0.058 | 25.593 | | 6.831 |
| 0.271 | 100.000 | 0.000 | 0.119 | 78.620 | | 6.875 | 0.052 | 18.763 | | 6.089 |
| 0.244 | 100.000 | 0.096 | 0.107 | 71.745 | | 7.400 | 0.047 | 12.674 | | 5.162 |
| 0.220 | 99.904 | 0.825 | 0.097 | 64.345 | | 7.756 | 0.042 | 7.512 | | 4.059 |
| 0.199 | 99.079 | 1.719 | 0.087 | 56.589 | | 7.932 | 0.038 | 3.453 | | 2.739 |
| 0.179 | 97.360 | 3.066 | 0.079 | 48.657 | | 7.932 | 0.034 | 0.714 | | 0.714 |
| 0.162 | 94.294 | 4.184 | 0.071 | 40.725 | | 7.748 | 0.031 | 0.000 | | 0.000 |
| 0.146 | 90.109 | 5.305 | 0.064 | 32.978 | | 7.384 | 0.028 | 0.000 | | 0.000 |

### <Preparation of Vehicle Eyedrop>

The vehicle described in <Preparation of Fibrate-Containing Eyedrop> was prepared and used as a vehicle eyedrop.

### [Eyedrop Administration Test]

Using 6-week-old female diabetic model mice (C57BL/6J db/db), an eyedrop administration test was performed as shown in Table 2. The eyedrop was applied twice a day, morning and evening.

### [Ocular Blood Flow Measurement Test]

Ocular blood flow measurements were performed under isoflurane inhalation anesthesia (induction 3.5 to 4.0%/min, maintenance 1.5 to 2.5%/min). The mice were kept at 36°C to 37°C with a heat insulation pad to perform experiments. Measurement of each evaluation item was started from 8 weeks of age. Measurements were taken every other week.

**[Table 2]**

| | Treatment group | Control group |
|---|---|---|
| Eyedrop | Fibrate nanoparticle-containing eyedrop | Vehicle eyedrop |
| Number of mice for administration | 6 mice | 6 mice |
| Frequency of eyedrop administration | Twice, morning and evening + 1 hour before measurement | Twice, morning and evening + 1 hour before measurement |
| Site where eyedrop is applied | Left eye | Left eye |

### <Measurement of Clinic Parameters>

As systemic parameters, a body weight, a casual blood glucose level, an intraocular pressure (left eye), a mean blood pressure, and an ocular perfusion pressure were measured every other week from 8 weeks of age. Intergroup analysis between the treatment group and the control group was performed by a Two-way Repeated Measured ANOVA Sidak's multiple test. Age analysis was performed by a Johckheere-Terpstra test.

The results are shown in FIGS. 3A to 4B (mean ± SEM).

The body weight tended to increase with age in both the treatment group and the control group. There was no significant difference in body weight gain between the treatment group and the control group (FIG. 3A).

Casual blood glucose levels remained high in both the treatment group and the control group. The casual blood glucose was not significantly different between weeks of age in either group (FIG. 3B).

The intraocular pressure (FIG. 3C), the mean blood pressure (FIG. 4A), and the ocular perfusion pressure (FIG. 4B) were not significantly different between the treatment group and the control group. There was no significant difference between weeks of age in any group.

### <Measurement of Baseline Optic Nerve Head Blood Flow>

The baseline optic nerve head (ONH) blood flow was measured every other week from 8 weeks of age, using a laser speckle blood flow measuring device (LSFG-Micro, Softcare Co., Ltd.). Measurements were performed on the left eye. Intergroup analysis between the treatment group and the control group was performed by Two-way Repeated Measured ANOVA. Age analysis was performed by a Johckheere-Terpstra test.

The results are shown in FIG. 5 (mean ± SEM). There was no significant difference in resting blood flow before stimulation between the treatment group and the control group. There was no significant difference between weeks of age in any group.

### <Measurement of Change in Ocular Blood Flow Due to Flicker Stimulation>

Measurement of a change in ocular blood flow due to flicker stimulation was performed every other week from 8 weeks of age. Flicker stimulation was performed at a frequency of 12 Hz for 3 minutes, and ocular blood flow measurement was performed every 20 seconds using LSFG-Micro. Measurements were performed on the left eye. Intergroup analysis between the treatment group and the control group was performed by a Two-way Repeated Measured ANOVA Sidak's multiple test.

The results are shown in FIGS. 6 and 7 (mean ± SEM). Each graph in FIG. 6 shows each of changes in blood flow after flicker stimulation at 8 weeks, 10 weeks, 14 weeks, and 12 weeks of age. FIG. 7 is a graph summarizing changes in total blood flow at each week of age. The "Base" on the horizontal axis of FIG. 6 indicates a baseline optic nerve head blood flow before stress.

In the control group, it was confirmed that the increased blood flow response after the flicker stimulation was disturbed. On the other hand, in the treatment group, the increased blood flow response after the flicker stimulation was not disturbed and was maintained. After 10 weeks of age, there was a significant difference in changes in blood flow after the flicker stimulation between the treatment group and the control group. From this result, it was confirmed that retinal neurovascular coupling disorder can be avoided by administration of the fibrate nanoparticle-containing eyedrop.

### <Measurement of Change in Ocular Blood Flow Due to Hyperoxia Stimulation>

Changes in ocular blood flow due to hyperoxia were measured every other week from 8 weeks of age. Flicker stimulation was performed at a frequency of 12 Hz for 3 minutes and measurements were taken every 20 seconds using LSFG-Micro. Measurements were performed on the left eye. Intergroup analysis between the treatment group and the control group was performed by a Two-way Repeated Measured ANOVA Sidak's multiple test.

The results are shown in FIGS. 8 and 9 (mean ± SEM). Each graph in FIG. 8 shows each of changes in blood flow after hyperoxia stimulation at 8 weeks, 10 weeks, 14 weeks, and 12 weeks of age. FIG. 9 is a graph summarizing changes in total blood flow at each week of age. The "Base" on the horizontal axis of FIG. 8 indicates a baseline optic nerve head blood flow before stress.

In the control group, it was confirmed that the hypoperfusion response after the hyperoxia stimulation was disturbed. On the other hand, in the treatment group, the hypoperfusion response after the hyperoxia stimulation was not disturbed and was maintained. From these results, it was confirmed that a damage to glial cells and vascular endothelial cells can be avoided by administration of the fibrate nanoparticle-containing eyedrop.

### <Electroretinogram (ERG)>

Electroretinogram examinations were performed every other week from 8 weeks of age. The examination was performed on the left eye. Intergroup analysis between the treatment group and the control group was performed by a Two-way Repeated Measured ANOVA Sidak's multiple test.

The results are shown in FIG. 10 (mean ± SEM). In the treatment group, the prolongation of b-wave latency observed in the control group was ameliorated. After 12 weeks of age, the b-wave latency in the treatment group was significantly shortened, as compared to the control group. From this result, it was confirmed that retinal dysfunction can be suppressed by administration of the fibrate nanoparticle-containing eyedrop.

### [Industrial Applicability]

According to the present invention, there is provided an eyedrop capable of ameliorating or preventing retinal circulation disorder and retinal neurovascular coupling disorder.

While preferred embodiments of the present invention have been described and illustrated above, it should be understood that these are exemplary of the invention and are not to be considered as limiting. Additions, omissions, substitutions, and other modifications can be made without departing from the spirit or scope of the present invention. Accordingly, the present invention is not to be considered as being limited by the foregoing description, and is only limited by the scope of the appended claims.

## Claims

1. An eyedrop for ameliorating or preventing retinal circulation disorder and a retinal neurovascular coupling disorder, the eyedrop comprising nanoparticles including a fibrate.

2. The eyedrop according to Claim 1,
wherein the nanoparticles are a wet-ground product of a fibrate.

3. The eyedrop according to Claim 1 or 2, further comprising a thickener.

4. The eyedrop according to Claim 3,
wherein the thickener is a cellulose-based thickener.

5. The eyedrop according to Claim 4,
wherein the cellulose-based thickener is methylcellulose.

6. The eyedrop according to any one of Claims 1 to 5,
further comprising cyclodextrins.

7. The eyedrop according to Claim 6,
wherein the cyclodextrins are 2-hydroxypropyl-β-cyclodextrin.
